# EUROPEAN PATENT APPLICATION

(11) **EP 4 691 545 A1**
(43) Date of publication of application: **11.02.2026**
(21) Application number: 24780287.9
(22) Date of filing: 26.03.2024
(51) Int. Cl.: A61N 1/30, A61N 1/04

(54) **TRANSDERMAL CURRENT-CARRYING PATCH**

(30) Priority: 28.03.2023 JP 2023052213
(71) Applicant: Kikkoman Corporation, Noda-shi, Chiba 278-8601 (JP)
(72) Inventor: MASAKARI, Yosuke, Noda-shi, Chiba 278-8601 (JP)
(74) Representative: J A Kemp LLP
(86) International application number: PCT/JP2024/011909
(87) International publication number: WO 2024/204174

(57) **Abstract**

The object is to provide a current-carrying patch that allows a drug to be permeated into the skin and a method for transdermal permeation of a drug. This invention provides a transdermal current-carrying patch comprising an anode electrode, an insulator, a cathode electrode, and a drug layer, where the drug placed in a part on the anode electrode side to be brought into contact with the skin is identical to the drug placed in a part on the cathode electrode side to be brought into contact with the skin, and a method of iontophoresis using this.

## Description

### Technical Field

The present invention relates to a patch utilizing iontophoresis, such as a transdermal current-carrying patch.

### Background Art

In the medical and cosmetic fields, iontophoresis technology that uses an electric current to allow a target component to be permeated into the skin has been known. Iontophoresis is a process comprising applying an electric potential to the skin to administer a drug transdermally.

In iontophoresis, an electric current can be applied as an alternating current or direct current. In the case of an alternating current, damage to the skin can be reduced, and an electric current can be applied in a milliampere unit per unit area. In the case of a direct current, an electric current of approximately 80 to 500 µA/cm² is often applied. If an electric current exceeds 500 µA/cm², it may irritate or damage the skin.

Iontophoresis devices are roughly classified into: a separate-type device comprising electrodes separately from pads; and an integrated-type device in which electrodes are integrated with pads. When an alternating current is applied as the electric current, a larger current can be applied. However, the device must be a separate-type device, and this is generally large.

Patent Literature 1 and Non Patent Literature 1 each describe a kit for adhesion to body tissue and a patch for adhesion to body tissue.

Patent Literature 2 describes an iontophoresis administration device. In the device having the structure shown in Figure 1 of Patent Literature 2, an electric current passes through in a drug-containing conductive layer provided on the skin, and the electric current does not substantially flow through the skin, and, as a consequence, effects of promoting permeation of an ionic compound into the skin cannot be expected.

Conventional iontophoresis technology is intended to facilitate permeation of an active ingredient into the skin by electric repulsion (see, for example, Patent Literature 3).

### Citation List

### Patent Literature

Patent Literature 1: JP 2014-207987 A
Patent Literature 2: WO 2021/078311 (counterpart: EP 4,049,714 A1)
Patent Literature 3: JP 2005-261970 A (Japanese Patent No. 4070779)

### Non Patent Literature

Non Patent Literature 1: ACS Appl. Electron. Mater., 2020, 2, 1, 170-176

### Summary of Invention

### Technical Problem

According to conventional iontophoresis technology, for example, a negatively-charged drug (an active ingredient) is placed on a cathode electrode, another active ingredient that is positively charged is placed on an anode electrode, and an electric current is applied, so as to facilitate permeation of the negatively-charged active ingredient on the cathode electrode by electric repulsion and facilitate permeation of the positively-charged active ingredient on the anode electrode by electric repulsion.

The present inventor recognized conventional iontophoresis technology in which an identical drug (the active ingredient) cannot be permeated into the skin from both electrodes as a technical problem to be addressed. For example, in order to allow a positively-charged drug to be permeated into the skin, only the anode electrode that is electrically repulsive to the drug can be used according to conventional techniques. In such a case, the cathode electrode would be a dead weight of the patch. In other words, according to conventional iontophoresis technology, the role of a patch on one side that does not have any drug (i.e., an empty patch) is limited to application of an electric current between the anode electrode and the cathode electrode, and the drug cannot be administered through the empty patch, and thus the drug cannot be administered through the site to which the empty patch is adhered. That is, the necessity of adhesion of a patch incapable of drug permeation was a problem of conventional techniques. The situation in which an empty patch occupies half of the adhesion area in a limited adhesion site diminishes the efficiency of drug permeation. Further, e.g., when drug permeation is intended to the whole area of a particular site or symmetrically to the right and the left of the site, it is necessary to apply a drug-containing patch separately from an empty patch and such necessity was a problem when trying to efficiently deliver the drug to the site of interest. In order to avoid forming an empty patch according to conventional techniques, one might have considered placing charged drugs suitable for relevant electrodes separately on both electrodes. However, in actual settings, there may not be necessarily be two types of drugs to administer simultaneously. Further, even when there are two types of drugs to administer, it may not necessarily be the case that one is a positively-charged drug and the other is a negatively-charged drug.

Accordingly, it is an object of the present disclosure to provide a patch and a method that allows identical active ingredients to be permeated into the skin through both electrodes with regard to iontophoresis technology. To the best of the present inventor's knowledge, such approach has not yet been reported, and accordingly this is a novel technical problem.

### Solution to Problem

Under the circumstances above, the present inventor conducted concentrated studies in order to examine as to whether or not an identical active ingredient can be permeated into the skin through both electrodes with regard to iontophoresis technology. As a result, the present inventor discovered, surprisingly, that not only is permeation of the active ingredient into the skin facilitated by electric repulsion when the drug to be delivered was placed on an electrode charged with the same charge as the drug but also, surprisingly, that drug permeation into the skin is facilitated when the drug to be delivered was placed on an electrode with a charge opposite of the charge of the drug and completed the present invention encompassing this as one embodiment.

The present invention encompasses the following embodiments.
[1] A transdermal current-carrying patch to be brought into contact with the skin to allow a drug to be permeated into the skin,
   wherein the transdermal current-carrying patch comprises an electrode body, a conductive part, an adhesive layer, a separator, and a surface film,
   the electrode body comprises a first electrode and a second electrode,
   the conductive part comprises a first conductive layer and a second conductive layer,
   the separator covers the adhesive layer and is to be removed when using the transdermal current-carrying patch,
   the adhesive layer is for adhering the transdermal current-carrying patch to the target skin and insulates the first conductive layer from the second conductive layer,
   the first conductive layer comprises a first electrolyte, a first buffer, and a drug,
   the second conductive layer comprises a second electrolyte, a second buffer, and a drug, and
   the first electrode, the second electrode, and the conductive part are connected to a power supply,
   wherein, when the transdermal current-carrying patch is brought into contact with the skin, an electric potential is applied from the power supply to enable power distribution among the first electrode, the skin, and the second electrode, thereby allowing an electric current to flow, and
   wherein, the drug comprised in the first conductive layer is identical to the drug comprised in the second conductive layer.
[2] The transdermal current-carrying patch according to embodiment 1, wherein permeation of both the drug comprised in the first conductive layer and the drug comprised in the second conductive layer into the skin are facilitated, compared with the case where an electric current does not flow.
[3] The transdermal current-carrying patch according to embodiment 1 or 2, wherein the drug is a positively-charged drug.
[4] The transdermal current-carrying patch according to embodiment 1 or 2, wherein the drug is a negatively-charged drug.
[5] A screening method to identify an identical drug that can be permeated into the skin through both the cathode electrode and the anode electrode by using a transdermal current-carrying patch, said screening method comprising:
   (i) a step of placing three-dimensional skin cells on a semipermeable membrane, placing a drug-containing solution on the three-dimensional skin cells, placing a first electrode on the three-dimensional skin cells, and placing a second electrode under or within the semipermeable membrane;
   (ii) a step of applying an electric potential at a constant electric current in the first direction and evaluating permeation of a drug into the three-dimensional skin cells;
   (iii) a step of applying an electric potential at a constant electric current in the second direction and evaluating permeation of a drug into the three-dimensional skin cells; and
   (iv) when permeation of the drugs into the three-dimensional skin cells is facilitated upon application of the electric potential both in the first direction and in the second direction, a step of identifying the drug as an identical drug that can be permeated into the skin through both the cathode electrode and the anode electrode by using the transdermal current-carrying patch,
      wherein, when permeation of a drug into the skin is enhanced upon application of the electric potential in the first direction compared with the case without application of the electric potential, permeation of the drug into the skin is evaluated as being facilitated, and
      when permeation of a drug into the skin is enhanced upon application of the electric potential in the second direction compared with the case without application of the electric potential, permeation of the drug into the skin is evaluated as being facilitated.
[6] A method for producing a transdermal current-carrying patch comprising:
   (i) a step of placing three-dimensional skin cells on a semipermeable membrane, placing a drug-containing solution on the three-dimensional skin cells, placing a first electrode on the three-dimensional skin cells, and placing a second electrode under or within the semipermeable membrane;
   (ii) a step of applying an electric potential at a constant electric current in the first direction and evaluating permeation of a drug into the three-dimensional skin cells;
   (iii) a step of applying an electric potential at a constant electric current in the second direction and evaluating permeation of a drug into the three-dimensional skin cells;
   (iv) when permeation of the drug into the three-dimensional skin cells is facilitated upon application of the electric potential both in the first direction and in the second direction, a step of identifying the drug as an identical drug that can be permeate into the skin through both the cathode electrode and the anode electrode by using the transdermal current-carrying patch; and
   (v) a step of incorporating the drug identified in step (iv) into the transdermal current-carrying patch.

The present description incorporates the contents disclosed by JP Patent Application No. 2023-052213, which is the base for priority of the present application.

### Advantageous Effects of Invention

According to the present invention, an identical drug can be permeated into the skin via both electrodes of a direct-current type transdermal current-carrying patch.

### Brief Description of Drawings

[Figure 1] Figure 1 shows an exploded perspective view of the transdermal current-carrying patch according to an embodiment of the present disclosure. The transdermal current-carrying patch 1 shown in Figure 1 comprises an electrode body 10 (a plurality of electrodes), two conductive parts 20 (a conductive layer and a plurality of conductive parts), an adhesive layer 30, a separator 40, and a surface film 50.
[Figure 2] Figure 2 shows a schematic view demonstrating the relationship between a catalyst (enzyme) 14 and an electron transfer mediator 15 in the anode electrode 11 according to an embodiment of the transdermal current-carrying patch shown in Figure 1.
[Figure 3] Figure 3 shows the relationship between the electric current density (µA/cm²) and the elapsed time (min) of the electric current flowing in the electric circuit formed by the transdermal current-carrying patch 1.

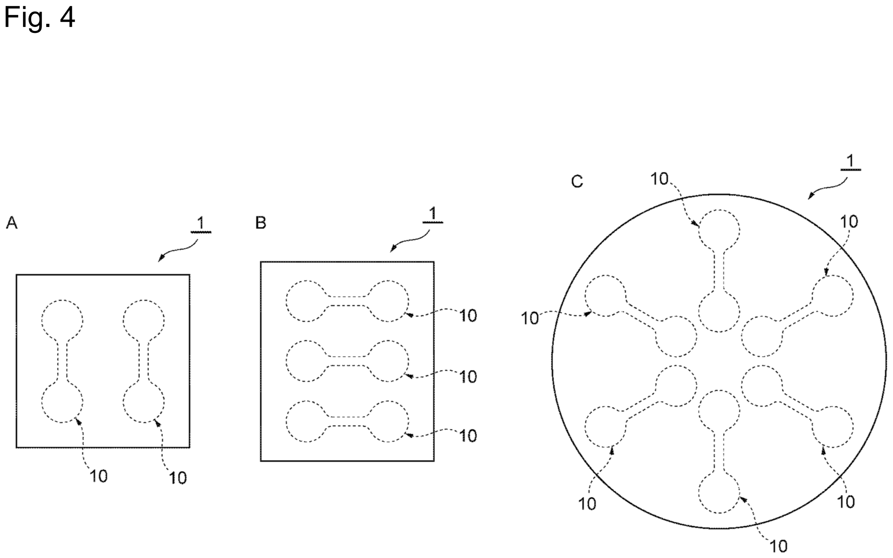

[Figure 4] Figure 4 shows a transdermal current-carrying patch according to an embodiment of the present disclosure.

### Description of Embodiments

In an embodiment, the present disclosure provides a transdermal current-carrying patch that is brought into contact with the skin to allow a drug to be permeated into the skin. The transdermal current-carrying patch comprises an electrode body, a conductive part, an adhesive layer, a separator, and a surface film. The electrode body comprises a first electrode and a second electrode. The conductive part comprises a first conductive layer and a second conductive layer. The separator covers the adhesive layer and is to be removed when the transdermal current-carrying patch is used. The adhesive layer is for adhering the transdermal current-carrying patch to the target skin and insulates the first conductive layer from the second conductive layer. The first conductive layer comprises a first electrolyte, a first buffer, and a drug. The second conductive layer comprises a second electrolyte, a second buffer, and a drug. The first electrode, the second electrode, and the conductive part are connected to a power supply. When the transdermal current-carrying patch is brought into contact with the skin, an electric potential is applied from the power supply to enable power distribution among the first electrode, the skin, and the second electrode, and an electric current flows. The drug comprised in the first conductive layer is identical to the drug comprised in the second conductive layer. The step of incorporating the drug into the conductive layer may be performed during the process of producing the transdermal current-carrying patch or a transdermal current-carrying patch produced without incorporating the drug into the conductive layer may be subjected to a step of incorporating the drug into the conductive layer before use. Alternatively, the drug may be applied to the skin in advance, or a conductive layer not containing a drug may be brought into contact with the skin, and the drug may be incorporated into part of the conductive layer as a consequence. The power supply may be an external power supply or an internal power supply, and it may be a battery or a fuel cell. In an embodiment, the power supply is an external power supply. In an embodiment, the power supply is an internal power supply. In an embodiment, the power supply is a battery. Examples of batteries include a button battery, a small type battery, and a thin type battery. In an embodiment, the power supply is a fuel cell. The transdermal current-carrying patch may comprise a wire and/or a lead. The wire may be included in the battery. The lead can connect the first electrode to the second electrode.

In an embodiment, the transdermal current-carrying patch of the present disclosure facilitates permeation of both the drug comprised in the first conductive layer and the drug comprised in the second conductive layer into the skin, compared with the case where an electric current is not flowing. In order to facilitate drug permeation using iontophoresis, it is necessary to allow an electric current to flow at a constant level. For convenience of description, in the present specification, the lower limit of the electric current that is necessary for drug permeation is designated to be 0.001 nA/cm². In one embodiment, even if an electric current is allowed to flow in the order of picoampere (pA) per 1 cm², an electric current is not considered as flowing from the transdermal current-carrying patch. For example, when a micro electric current (low electric current) of approximately 0.1 pA/cm² is allowed to flow, it is considered that an electric current does not flow.

The first electrode is designated as an anode electrode herein for the convenience of description. Further, the second electrode is designated as a cathode electrode herein for the convenience of description. The anode electrode may comprise an enzyme, carbon, or metal. In an embodiment, the anode electrode can be an enzyme electrode. In an embodiment, the anode electrode can be a metal electrode. The cathode electrode comprises a substance that catalyzes a reducing reaction, such as an enzyme, organic complex, or metal. In an embodiment, the cathode electrode can be an enzyme electrode. In an embodiment, the cathode electrode can be a metal electrode. The above description is provided for the convenience of description, and such description does not indicate that the enzyme electrode is metal-free. An example of an organic complex is a transition metal complex, such as iron phthalocyanine. The enzyme electrode is not particularly limited, provided that an enzyme used therefor is involved in electron transmission and an enzyme immobilized on an electrode material, such as metal, falls under the category of the enzyme electrode. In an embodiment, the first electrode (anode electrode) is an enzyme electrode, and the second electrode (cathode electrode) is an enzyme electrode. In an embodiment, the first electrode (anode electrode) is an enzyme electrode, and the second electrode (cathode electrode) is a metal electrode. In an embodiment, the first electrode (anode electrode) is a metal electrode, and the second electrode (cathode electrode) is an enzyme electrode. In an embodiment, the first electrode (anode electrode) is a metal electrode, and the second electrode (cathode electrode) is a metal electrode.

In one embodiment, when an enzyme electrode is used as the first electrode or the second electrode, the first conductive layer or the second conductive layer corresponding thereto may comprise an absorptive body. When the first conductive layer comprises the absorptive body, such first conductive layer is referred to as the "first absorptive body" for the convenience of description. Further, when the second conductive layer comprises the absorptive body, such second conductive layer is referred to as the "second absorptive body" for the convenience of description. When the first conductive layer comprises the first absorptive body and/or the second conductive layer comprises the second absorptive body, such absorptive body may comprise a first electrolyte, a first buffer, and a drug. When a second absorptive body is present, it may comprise a second electrolyte, a second buffer, and a drug. In an embodiment, the first absorptive body may comprise a first fuel. The first fuel may be any substance, provided that such substance can serve as a fuel (substrate) of the first oxidoreductase of the first electrode. In an embodiment, the second absorptive body may comprise a second fuel. The second fuel may be any substance, provided that such substance can serve as a fuel (substrate) of the second oxidoreductase of the second electrode.

In an embodiment, the transdermal current-carrying patch comprises a fuel cell. In an embodiment, the transdermal current-carrying patch comprises a fuel cell, the first electrode is an enzyme electrode comprising a first oxidoreductase, the first conductive layer comprises a first absorptive body, and the first absorptive body comprises a first fuel. In an embodiment, the transdermal current-carrying patch comprises a fuel cell, the second electrode is an enzyme electrode comprising a second oxidoreductase, the second conductive layer comprises a second absorptive body, and the second absorptive body comprises a second fuel. In an embodiment, the transdermal current-carrying patch comprises a fuel cell, the first electrode is an enzyme electrode comprising a first oxidoreductase, the first conductive layer comprises a first absorptive body, the first absorptive body comprises a first fuel, the second electrode is an enzyme electrode comprising a second oxidoreductase, the second conductive layer comprises a second absorptive body, and the second absorptive body comprises a second fuel. The fuel cell is configured such that when the transdermal current-carrying patch is brought into contact with the skin, the skin moisture is absorbed by the absorptive body, the electrolyte comprised in the absorptive body is dissolved, power distribution between the first electrode and the second electrode becomes possible, and an electric current then flows.

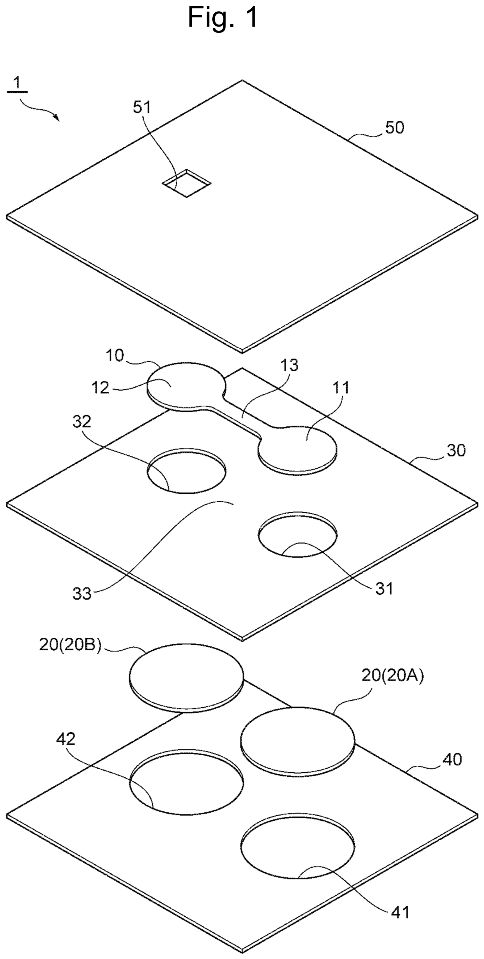

In an embodiment, the present disclosure provides the transdermal current-carrying patch shown in Figure 1. The transdermal current-carrying patch 1 is an electric current patch that makes use of a biobattery using an enzyme. The transdermal current-carrying patch 1 may comprise an electrode body 10 (a plurality of electrodes), two conductive parts 20 (a conductive layer and a plurality of conductive parts), an adhesive layer 30, a separator 40, and a surface film 50. When using the transdermal current-carrying patch 1, the separator 40 is removed, and the patch is adhered to the skin of the subject (user) through the adhesive layer 30. The patch can be adhered to an appropriate site of the skin, such as the shoulder, arm, or chin. The patch can be appropriately shaped for easy adhesion to the target site. Upon adhesion of the transdermal current-carrying patch 1, each electrode in the electrode body 10 is brought into contact with the site of the subject through the conductive parts 20, and an electric circuit that allows a micro electric current to flow is formed. A transdermal current-carrying patch may also be referred to as an "iontophoresis patch" or a "patch for iontophoresis."

In an embodiment, an electric current flowing through the electric circuit to the site of the subject and the proximal region thereof can be, for example, a direct electric current having the electric current density of 0.001 nA/cm² to less than 500 µA/cm². In an embodiment, the transdermal current-carrying patch 1 can be configured so as to generate an electric current having the electric current density lower than 500 µA/cm², which is a threshold above which the subject senses irritation. A micro electric current that is allowed by the transdermal current-carrying patch 1 to flow to the site of the subject can be, for example, 0.001 nA/cm² or more, 0.005 nA/cm² or more, 0.01 nA/cm² or more, 0.05 nA/cm² or more, 0.1 nA/cm² or more, 0.001 µA/cm² or more, 0.005 µA/cm² or more, 0.01 µA/cm² or more, 0.5 µA/cm² or more, 1 µA/cm² or more, 2 µA/cm² or more, 5 µA/cm² or more, 10 µA/cm² or more, 20 µA/cm² or more, 30 µA/cm² or more, or 40 µA/cm² or more to less than 50 µA/cm², less than 100 µA/cm², less than 200 µA/cm², less than 300 µA/cm², less than 400 µA/cm², or less than 500 µA/cm².

The electrode body 10 comprises an anode electrode 11 (negative electrode), a cathode electrode 12 (positive electrode), and a lead 13 (connecting part). The lead 13 connects the anode electrode 11 to the cathode electrode 12. The anode electrode 11, the lead 13, and the cathode electrode 12 are arranged in this order and may be formed as an integrated member. The thickness of the electrode body 10 can be, for example, approximately 0.1 mm to 2.0 mm. The size of the transdermal current-carrying patch 1 can be, for example, a width of approximately 1 cm to 10 cm and a length of approximately 1 cm to 10 cm. The size (area) of the electrode body 10 in the transdermal current-carrying patch 1 is smaller than that of the entire transdermal current-carrying patch 1, and the size (area) of the anode electrode 11 and that of the cathode electrode 12 can be appropriately modified in terms of the geometrical surface area in accordance with the site of adhesion and the range in which a micro electric current is to be allowed to flow and, for example, can be 0.1 cm² or larger, 0.5 cm² or larger, 1 cm² or larger, 3 cm² or larger, 5 cm² or larger, 10 cm² or larger, 20 cm² or larger, 30 cm² or larger, 40 cm² or larger, or 50 cm² or larger, such as 80 cm² or larger to 100 cm² or smaller, 80 cm² or smaller, 50 cm² or smaller, 40 cm² or smaller, 30 cm² or smaller, 20 cm² or smaller, 10 cm² or smaller, 5 cm² or smaller, 3 cm² or smaller, 1 cm² or smaller, or 0.5 cm² or smaller, such as 0.1 cm² or smaller. To a target site such as a sore spot, one or a plurality of the transdermal current-carrying patches 1 can be applied. The number of the electrode body 10 is not limited to one, and the transdermal current-carrying patch 1 may comprise one or a plurality of the electrode bodies 10. The form of the transdermal current-carrying patch 1 is not particularly limited, and the transdermal current-carrying patch 1 can be in an appropriate form, such as a polygonal, hexagonal, pentagonal, tetragonal, trigonal, circular, or ellipsoidal form.

Examples of materials constituting the anode electrode 11, the cathode electrode 12, and the lead 13 include: carbon materials, such as carbon nanotubes, Ketjenblack^{®}, Glassy carbon^{®}, graphene, fullerene, carbon fiber, carbon fabric, and carbon aerogel; conductive polymers, such as polyaniline, polyacetylene, polypyrrole, poly(p-phenylenevinylene), polythiophene, and poly(p-phenylenesulfide); semiconductors, such as silicone, germanium, indium tin oxide (ITO), titanium oxide, copper oxide, and silver oxide; and metals, such as gold, platinum, titanium, aluminum, tungsten, copper, silver, zinc, magnesium, iron, and palladium. From the viewpoint of flexibility and electrochemical stability, in particular, a material constituting the electrode body 10 is preferably a carbon material, such as a carbon fabric or carbon nanotube. When an enzyme is immobilized on the electrode at a high concentration, in particular, the material constituting the electrode body 10 is preferably a carbon nanotubemodified carbon fabric. A paste material may also be used as an electrode material. For example, a mixture of a carbon paste and an electrode paste material may be used, or an electrode paste material may be superposed only on necessary parts on a carbon material.

The anode electrode 11 may comprise a catalyst that catalyzes the oxidation reaction supported thereon. Examples of catalysts include oxidoreductases, such as glucose oxidase, glucose dehydrogenase (GDH), D-fructose dehydrogenase (FDH), alcohol oxidase, alcohol dehydrogenase, lactate oxidase, and lactate dehydrogenase and the like. Examples of materials other than enzymes include magnesium, a magnesium-containing alloy, aluminum, an aluminum-containing alloy, calcium, iron, and zinc and the like, and an electrode composed of at least one of such materials can be used.

In an embodiment, an electron transfer mediator can be immobilized on an electrode. Examples of electron transfer mediators that can be used include known mediators, modified products, and derivatives thereof. Examples of electron transfer mediators include phenazines, viologens, cytochromes (e.g., cytochrome b and cytochrome c), phenoxazines, phenothiazines, ferricyanide such as potassium ferricyanide, ferredoxins, ferrocenes, a quinone-based compound, a phenylenediamine compound, an osmium complex, and a derivative of any thereof and the like, and examples of phenazine compounds include, but are not limited to, mediators such as phenazine methosulfate (PMS) and methoxy-PMS. Preferable examples of quinone-based compounds used as mediators include 1,4-naphthoquinone, 1,2-naphthoquinone, and 2-methyl-1,4-naphthoquinone. Examples of phenylenediamine-based compounds include N-isopropyl-N'-phenyl-p-phenylenediamine (IPPD), N,N'-diphenyl-p-phenylenediamine (DPPD), and N-(1,3-dimethylbutyl)-N'-phenyl-p-phenylenediamine (6PPD). The compounds described in WO 2019/198359 may be used. With the use of an electron transfer mediator, an electric current in the electric circuit can be enhanced to the range described above, when the transdermal current-carrying patch 1 is applied to a given site of the subject.

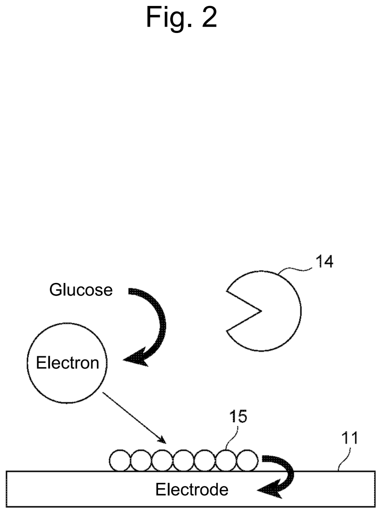

For example, the anode electrode 11 shown in Figure 2 comprises, immobilized thereon, an electron transfer mediator 15 that facilitates electron transfer between the electrode (anode electrode 11) and the enzyme 14 that functions as a catalyst in a biobattery. At the anode electrode 11, an electron can be efficiently taken from, for example, glucose as a fuel, with the aid of the enzyme 14 and the electron transfer mediator 15 immobilized on the electrode.

In an embodiment, the cathode electrode may comprise a catalyst that catalyzes the reduction reaction supported thereon. Examples of catalysts include: enzymes such as bilirubin oxidase (BOD), laccase, Cu efflux oxidase (Cueo), ascorbate oxidase, glucose oxidase, lactate oxidase, amino acid oxidase, sarcosine oxidase, and peroxidase; transition metal complexes such as iron(II) phthalocyanine; platinum; and metal oxides composed of at least one metal selected from among titanium, nickel, stainless steel, iron, manganese, zinc, copper, and molybdenum or at least one metal selected from among calcium, iron, manganese, zinc, copper, and molybdenum. See, for example, the cathode electrode 12 shown in Figure 2.

The conductive part 20 is an absorptive body that is placed to be in surface contact with the anode electrode 11 and the cathode electrode 12. The conductive part 20 comprises a dry fuel or electrolyte embedded inside a sponge or gel. The first conductive layer 20A that is in contact with the anode electrode 11 comprises a fuel, such as an organic matter and the like, that causes an oxidation reaction at the anode electrode 11. Examples of fuels include glucose, fructose, ascorbic acid (vitamin C), alcohol, and lactic acid and the like (also see Figure 2).

An absorptive body constituting the conductive part 20 comprises a buffer. A buffer is an electrolyte that serves as a buffer when prepared in the form of an aqueous solution. Examples of buffers include salts such as weak acids and weak bases. An absorptive body may comprise or need not comprise an electrolyte other than a buffer, such as a salt with a strong acid or strong base. Examples of electrolytes constituting a buffer include: weak acids such as phosphoric acid, acetic acid, citric acid, and tartaric acid and the like; a sodium salt and potassium salt and the like of such weak acids; weak bases such as organic amine, and the like and salts thereof and the like. Examples of electrolytes include, but are not limited to, acetic acid, sodium acetate, potassium acetate, citric acid, sodium citrate, calcium citrate, phosphoric acid, disodium hydrogen phosphate, sodium dihydrogen phosphate, polyphosphoric acid, glucosamine hydrochloride, monoethanolamine, diethanolamine, triethanolamine, trometamol, meglumine, potassium chloride, sodium chloride, calcium chloride, lactic acid, sodium lactate, potassium lactate, hydrochloric acid, triethanolamine hydrochloride, sodium hydroxide, calcium hydroxide, potassium hydroxide, cation-exchange resin having an acetic acid group or sulfone group as a functional group, anion-exchange resin having a quaternary ammonium group as a functional group or a salt thereof, primary to tertiary amines or a polymer or resin comprising the same, cholestyramine, acidic amino acids such as aspartic acid and glutamic acid, basic amino acids such as lysine, histidine, and arginine, other amino acids such as arginine hydrochloride, glutamine, and sodium glutamate and the like, and polyamines and the like. A buffer may be composed of two or more electrolytes. When an absorptive body does not comprise a buffer, water to be absorbed by an absorptive body may comprise a buffer. Alternatively, both an absorptive body and water to be absorbed thereby may comprise a buffer. Optionally, an absorptive body may be supplemented with a stabilizer, a thickener, a wetting agent, a surfactant, a solubilizing agent (solubilizer), a solubilization supporting agent, a moisturizing agent, or an absorption promoter.

Examples of surfactants or solubilization supporting agents include, but are not limited to: polyoxyethylene compounds, such as polyoxyethylene sorbitan monooleate, polyoxyethylene sorbitan monolaurate, polyoxyethylene lauryl ether, polyoxyethylene octylphenyl ether, polyoxyethylene nonylphenyl ether, polyoxyethylene polyoxypropylene glycol, polyoxyethylene hydrogenated castor oil 60, polysorbate, and sucrose fatty acid ester; saccharides, such as glucose, maltose, fructose, galactose, mannitol, sorbitol, mannose, glucosamine, lactose, sucrose, and trehalose and the like; water-soluble cyclodextrins, such as natural cyclodextrin, such as α-cyclodextrin, β-cyclodextrin, or γ-cyclodextrin and the like, or a water-soluble cyclodextrin derivative with a substituent, such as a hydroxypropyl, glycolyl, maltosyl, sulfuric acid, phosphoric acid, carboxyl, carboxymethyl, carboxymethylethyl, or amino group bound and the like thereto, and cyclodextrin polymers; water-soluble polymers, such as starch, dextran, dextran sulfate, inulin, and polyvinyl pyrrolidone and the like; and wetting agents, such as glycerin, ethylene glycol, polyethylene glycol, propylene glycol, butylene glycol, urea, ethylurea, an urea derivative, methyl pyrrolidone, and a pyrrolidone derivative and the like.

After the transdermal current-carrying patch 1 is produced, unless specified otherwise, the absorptive body of the conductive part 20 is kept dry before use. When using the transdermal current-carrying patch 1, water is supplied to the transdermal current-carrying patch 1, the absorptive body absorbs water, and an electrolytic solution comprising an electrolyte is then embedded within the absorptive body. Thus, the anode electrode 11 and the cathode electrode 12 become electrically connected to the skin through the electrolytic solution to form an ion transfer pathway including the anode electrode 11, the first conductive layer 20A, the skin, the second conductive layer 20B, and the cathode electrode 12. For example, cations such as hydrogen ions and sodium ions and the like are transported from the anode electrode 11 toward the cathode electrode 12.

The absorptive body of the conductive part 20 may comprise a gel or sponge. Examples of gels include: hydrophilic and conductive gels, such as polymers including a methoxyethylene-maleic anhydride copolymer, a methoxyethylene-maleic acid copolymer, polyacrylic acid, sodium polyacrylate, an isobutylene-maleic anhydride copolymer, an isobutylene-maleic acid copolymer, an N-vinyl acetamide-sodium acrylate copolymer, a carboxylvinyl polymer, polyurethane, polyvinyl pyrrolidone, polyvinyl alcohol, and polyacrylamide and the like; polysaccharides and gelatins, such as cellulose, agar, starch, mannan, xanthane gum, locust bean gum, carrageenan, gellan gum, tamarind gum, curdlan, pectin, furcellaran, guar gum, alginic acid, sodium alginate, tara gum, karaya gum, gum Arabic, hydroxypropyl cellulose, carboxymethyl cellulose, ethyl cellulose, ethyl methyl cellulose, and derivatives thereof; and gelatin and the like and combinations thereof. Examples of sponge materials include, but are not limited to; synthetic resin, such as polyurethane and polyvinyl alcohol and the like; and naturally-occurring polymers, such as cellulose and the like. A sponge can be an organic porous body. Examples of organic porous bodies include, but are not limited to, fiber aggregates formed of cellulose, cellulose acetate, nitrocellulose, rayon, polyethylene, polypropylene, polyethylene terephthalate, nylon, polyester, and synthetic resin chemically modified therewith, paper, woven fabric, unwoven fabric, and porous synthetic resins, such as porous polypropylene, porous polystyrene, porous polymethyl methacrylate, porous polyester, porous nylon, porous polysulfone, porous fluorocarbon resin, porous polyurethane, and porous polyvinyl alcohol and the like.

In the absorptive body of the conductive part 20, a buffer can be embedded in a sponge having air bubbles or gel. Continuous fine bubbles may be formed within the sponge. Thus, the electrolyte as a solute within the sponge can be dried by drying the sponge that has absorbed an electrolytic solution consisting of an aqueous electrolyte solution. At least a part of the electrolyte can be exposed in a solid state on the inner wall surface of air bubbles without being incorporated into a sponge material. The sponge or gel may comprise a biobattery fuel, a drug that can act on an organism's body, other additives, and the like, in addition to the electrolyte.

The sponge of the conductive part 20 can have excellent water-absorbing properties because of capillary action, surface tension, hydrophilicity, and the like. This enables rapid water absorption by merely soaking a part of the sponge, such as the bottom surface, in water. In addition, a solute, such as an electrolyte, is dissolved in water in an inner space of sponge bubbles, and an electrolytic solution is thereby prepared. Because of the water-absorbing capacity of the sponge, the electrolytic solution is homogeneously mixed and spread to the entire absorptive body, and the anode electrode 11 and the cathode electrode 12 can be connected to the skin through the electrolytic solution. The absorptive body configured with the use of the sponge can transfer the moisture content in a direction against gravity or in a complicated form, such as in a three-dimensional form and the like.

A sponge constituting the conductive part 20 may comprise a large number of pores. A pore diameter can be, e.g., 10 µm to 500 µm, 10 µm or larger, 20 µm or larger, 30 µm or larger, 50 µm or larger, 80 µm or larger, 100 µm or larger, 120 µm or larger, 150 µm or larger, or 200 µm or larger, such as 300 µm or larger, to 500 µm or smaller, 300 µm or smaller, 200 µm or smaller, 150 µm or smaller, 120 µm or smaller, 100 µm or smaller, 80 µm or smaller, 50 µm or smaller, 30 µm or smaller, or 20 µm or smaller, such as 10 µm or smaller. Specific examples of pore diameters include, but are not limited to, 10 µm, 20 µm, 25 µm, 30 µm, 50 µm, 80 µm, 100 µm, 150 µm, 200 µm, 300 µm, 500 µm, intermediate values thereof, and proximate values thereof. The sponge porosity can be, for example, 60% to 95%, 65% to 90%, or 70% to 85%, such as 75% to 80%. A sponge is preferably a polyurethane sponge, while a sponge having water-absorbing properties and other properties as excellent as those of the polyurethane sponge may also preferably be used. As a sponge constituting the conductive part 20, for example, Sofras (product name, manufactured by Aion Co., Ltd.) can be used. Although the thickness of the sponge constituting the conductive part 20 can be approximately 0.1 mm to 3 mm, approximately 0.5 mm to 2 mm, approximately 0.8 mm to 1.8 mm, or approximately 1 mm to 1.6 mm, such as approximately 1.2 mm to 1.4 mm, it is possible to adjust the thickness of the sponge when integrated into the transdermal current-carrying patch 1 since a sponge has a large number of pores.

The transdermal current-carrying patch 1 utilizing a biobattery can use one or more types of enzyme electrodes as the anode electrode 11 or the cathode electrode 12. When the absorptive body of the conductive part 20 absorbs water, biobattery energization is initiated, and the transdermal current-carrying patch 1 is driven by the biobattery. While the absorptive body of the conductive parts 20 retains the electrolytic solution as a tank, it enables transfer of a material such as ions and fuels and the like from the anode electrode 11 or the cathode electrode 12 to the skin.

The adhesive layer 30 is a member for adhering the transdermal current-carrying patch 1 to the skin of the subject. The adhesive layer 30 can be composed of, for example, an insulating two-sided adhesive tape. The adhesive layer 30 is provided with 2 openings 31 and 32, the anode electrode 11 is accommodated in the opening 31, and the cathode electrode 12 is accommodated in the other opening 32. The lead 13 between the anode electrode 11 and the cathode electrode 12 is adhered to a part 33 (insulated part) between the opening 31 and the opening 32. With this, the position of the electrode body 10 relative to the adhesive layer 30 is fixed. In the adhesive layer 30, the anode electrode 11 accommodated in the opening 31 is brought into contact with the first conductive layer 20A, and the cathode electrode 12 accommodated in the opening 32 is brought into contact with the second conductive layer 20B. In such a case, the outer frame of the conductive layer 20A and that of the conductive layer 20B are fixed on the adhesive layer 30. Due to such configuration, the first conductive layer 20A is ionically insulated from the second conductive layer 20B. The adhesive layer 30 can have a thickness of, for example, approximately 0.1 mm to 0.5 mm, such as 0.2 mm to 0.4 mm.

The separator 40 is a member that works to ionically insulate the first conductive layer 20A from the second conductive layer 20B in combination with the adhesive layer 30. For example, the separator 40 can be formed of a silicone-coated release paper on the surface of a film or paper made of polyester or polyethylene terephthalate and the like. The separator 40 is provided with 2 openings 41 and 42, the first conductive layer 20A is accommodated in the opening 41, and the second conductive layer 20B is accommodated in the other opening 42. The separator 40 can have a thickness of, for example, approximately 0.05 mm to 0.1 mm, such as 0.06 mm to 0.08 mm.

The surface film 50 is a member that works to cover and protect the electrode body 10 and the conductive parts 20. The surface film 50 can be formed of, for example, a polyvinyl chloride film. When oxygen is used as a catalyst, a window part 51 may be formed at a position corresponding to the cathode electrode 12 of the surface film 50 in order to supply oxygen to the cathode electrode 12. In order to avoid exposure of the cathode electrode 12, a material that allows oxygen permeation, such as cotton, may be used for the window part 51. With this, the cathode electrode 12 can be protected while supplying oxygen.

The transdermal current-carrying patch 1 can be configured as a small and/or thin current-carrying patch, and it can readily remain adhered to a given site of the subject for a long period of time. When the transdermal current-carrying patch 1 is adhered to a given site of the subject after water absorption, the anode electrode 11 and the cathode electrode 12 are brought into contact with an organism's body through the first conductive layer 20A and the second conductive layer 20B, and an electric circuit that flows a micro electric current can be formed on the given site (including adjacent regions). The transdermal current-carrying patch 1 can be configured such that the electric circuit allows a micro electric current to flow in an organism's body, which is a direct electric current at an electric current density of 10 µA/cm² or higher when the resistance is 5 kΩ.

The electric resistance in the organism's body to which the transdermal current-carrying patch 1 is to be applied is explained. The electric resistance in the organism's body can be classified as the resistance on the skin or the resistance within a human body. The resistance of the skin varies depending on, for example, the wet conditions of the contact surface (see the Handbook for electric installation and safety and sanitation thereof, Vol. 4, the Ship's Electric Installation Contractors' Association of Japan). While resistance of the skin is approximately 10 kΩ on the dry and hardened skin, resistance of the skin is reduced to approximately one twelfth upon perspiration. Since the skin resistance is approximately 1 kΩ upon perspiration, the transdermal current-carrying patch 1 of the present embodiment can be configured to flow a direct electric current of 500 µA/cm² or lower when it is connected to a resistance of 1 kΩ. This can reduce the irritation sensed by the subject.

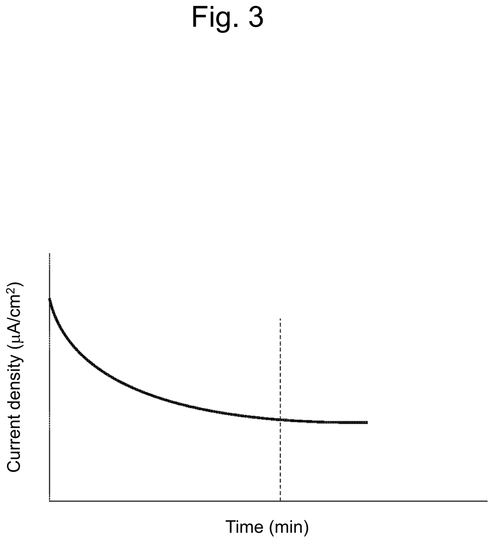

Figure 3 shows the relationship between the electric current density (µA/cm²) and the elapsed time (min) of the electric current flowing in the electric circuit configured by the transdermal current-carrying patch 1. This electric current density is that when the electric circuit of the transdermal current-carrying patch 1 is connected to a resistance of 10 kΩ. In Figure 3, while a relatively high electric current density is observed immediately after the initiation, the electric current density is reduced to the range of the micro electric current with the elapse of time. In an embodiment, an electric circuit formed by the transdermal current-carrying patch 1 is configured to allow a direct electric current with an electric current density of 10 µA/cm² to 100 µA/cm² to flow to a given site of the subject when connected to a resistance of 10 kΩ. In an embodiment, the transdermal current-carrying patch 1 is configured such that the electric circuit allows a micro electric current to flow at 10 µA/cm² to 175 µA/cm² to the given site at a time point after the elapse of a given time (e.g., 10 minutes at the latest) after the transdermal current-carrying patch is brought into contact with said given site of the subject. More specifically, the electric circuit of the transdermal current-carrying patch 1 is preferably configured such that a micro electric current flows at a current density of 10 µA/cm² to 175 µA/cm² at the time point up to 10 minutes after the connection to a resistance of 5 kΩ. In one embodiment, the transdermal current-carrying patch 1 may be configured such that the electric circuit can allow the electric current density of the micro electric current flowing at the time point that is at least 5 hours, such as at least 6 hours, after the transdermal current-carrying patch 1 is connected to a resistance of 5 kΩ, to be maintained at an electric current density of 10 µA/cm² to 175 µA/cm². The transdermal current-carrying patch 1 of the present embodiment may be adhered to the given site of the subject for a long period of time to continuously supply a micro electric current in a given range. In the present specification, if there is any uncertainty, a micro electric current that the transdermal current-carrying patch allows to flow to the site of application of the subject is defined as an electric current that is measured 6 hours after the patch is applied to the skin.

Figure 3 shows an example of an electric current density of the transdermal current-carrying patch 1. This is a chart demonstrating the electric current density measured using one sample of the transdermal current-carrying patch 1 that was actually produced. The transdermal current-carrying patch is configured to allow a micro electric current to flow to the given site of the subject at an electric current density maintained at 10 µA/cm² to 30 µA/cm² 10 minutes (600 seconds) after being connected to a resistance of 5 kΩ, and it is configured to allow a micro electric current to flow to the given site of the subject at an electric current density maintained at 10 µA/cm² to 30 µA/cm² at the time point after the elapse of at least 1 hour. By modifying the types and the amounts of catalysts or electron transfer mediators used for the transdermal current-carrying patch 1, the direct electric current flowing to the electric circuit of the transdermal current-carrying patch can be adjusted to the range described above, and the electric circuit may be configured to allow a direct electric current having an electric current density of 35 µA/cm² or more when it is connected to a resistance of 5 kΩ to flow, or it may be configured to allow a direct current having an electric current density of 60 µA/cm² or more to flow when connected to a resistance of 5 kΩ.

The conductive part 20; i.e., the conductive layer, may comprise a drug. As such, the conductive layer may be referred to as a "drug layer" in the present specification. Figure 1 shows the configuration of the transdermal current-carrying patch that comprises two conductive parts 20. However, when a plurality of the electrode bodies 10 are used, the conductive parts 20 corresponding to relevant electrode bodies 10 can be placed respectively. In the present disclosure, the first conductive layer 20A for the anode electrode and the second conductive layer 20B for the cathode electrode comprise the same drug component.

Microneedles may be connected to the conductive parts 20. Substrates of the microneedle may preferably be a polymer that can readily be injection-molded or press-molded, and examples thereof include nylon, polycarbonate, polylactic acid, a poly(lactic acid-glycolic acid) copolymer, polyglycolic acid, polyethylene terephthalate, a cyclic olefin polymer (COP), and mixtures thereof. Alternatively, any non-biodegradable polymer may be used without particular limitation, provided that, upon being molded into a microneedle, the same would not be completely dissolved for at least 15 minutes after being inserted into the skin, and examples thereof include hyaluronic acid, dextran, polyvinyl pyrrolidone, sodium chondroitin sulfate, hydroxypropyl cellulose, polyvinyl alcohol, and mixtures thereof. When the microneedle itself is conductive, the microneedle may be coated with a drug.

Any target drug can be comprised in the drug layer. Examples of drugs include, but are not limited to: topical anesthetics, such as lidocaine, epinephrine, dibucaine, tetracaine, oxybuprocaine, procaine, bupivacaine, and salts thereof; and physiologically active substances, such as antiinflammatory agents, analgesics, antipyretics, adenocorticotropic hormones, steroidal compounds, nonsteroidal compounds, ketoprofen, prostaglandins, and compounds integrated into cosmetic products and the like. Topical anesthetics may be integrated in the form of a salt, such as hydrochloride or sulfate and the like. Alternatively, when topical anesthetics are integrated in the form of a base, acidic additives, such as hydrochloric acid or sulfuric acid, may be added to the pharmaceutical preparation. The amount of the drug to be integrated (the amount to be retained) can be an effective amount in accordance with, for example, the type of drug, the site of administration, absorbability, biological efficacy, or a metabolic rate and the like. The amount of the drug per transdermal current-carrying patch can be, for example, 0.001 mg to 100 g, 0.005 mg to 10 g, 0.01 mg to 5 g, 0.05 mg to 1 g, or 0.1 mg to 500 mg, such as 1 mg to 100 mg. In one embodiment, the drug can be charged and for example, a drug can be positively or negatively charged. In other words, the present disclosure provides a transdermal current-carrying patch that allows a charged drug, such as a positively or negatively charged drug, to be permeated into the skin. In an embodiment, the drug can have polarity. In an embodiment, the drug can be hydrophobic. In an embodiment, the drug can have a molecular weight of 1000 Da or lower, such as 500 Da or lower.

Unless specified otherwise, the transdermal current-carrying patch 1 of the present disclosure is configured to allow a direct electric current to flow when it is adhered to the subject. However, when the transdermal current-carrying patch 1 of the present disclosure is adhered to the subject, direct electric current need not necessarily flow continuously at a given electric current density. When the transdermal current-carrying patch 1 of the present disclosure is adhered to the subject, typically, the moisture such as sweat from the skin is absorbed by the absorptive body, the electrolyte is dissolved, power distribution is enabled, and a direct electric current is allowed to flow. However, the electric current may be temporarily lowered or may stop flowing because of a change in the moisture content such as sweat. In the present disclosure, such temporary reduction or halt of the electric current is acceptable.

Unless specified otherwise, after the transdermal current-carrying patch of the present disclosure is adhered to an organism's body, the direction of the electric current is not reversed. In one embodiment, a transdermal current-carrying patch that reverses the direction of the electric current after adhesion thereof to an organism's body is excluded from the scope of the transdermal current-carrying patch of the present disclosure.

In an embodiment, an electric current may be applied as pulses (rectangular-shaped direct voltage pulses). A "pulsed current" can be an intermittent pulse, such that an electric current does not flow, which is designated as "0 V (zero volts)," an electric current then flows at a positive V value at a regular interval, and the electric current stops flowing again (becoming 0 V). Alternatively, an electric current does not flow (0 V), an electric current then flows at a negative V value at a regular interval, and the electric current stops flowing again (becoming 0 V). However, a pulsed current is not composed of a pulse of positive V followed by a pulse of negative V from the same electrode. Accordingly, in one embodiment, a patch driven by an alternating electric current is excluded from the scope of the transdermal current-carrying patch of the present disclosure. The frequency of the pulse can be selected from a range of 0.1 to 200 kHz or 1 to 200 kHz, such as 5 to 80 kHz. The on-off ratio of a pulsed direct voltage can be selected from a range of 1/10 to 20/1, or 1/50 to 15/1, such as 1/30 to 10/1. Energization time in the case of continuous energization can be 72 hours or shorter, 60 hours or shorter, 48 hours or shorter, 36 hours or shorter, or 24 hours or shorter to 1 hour or longer, 2 hours or longer, 3 hours or longer, 4 hours or longer, 6 hours or longer, 8 hours or longer, or 12 hours or longer, such as 1 to 72 hours, 2 to 48 hours, or 3 to 24 hours.

In an embodiment, an electric current may be allowed to flow with the application of a positive or negative voltage using a sine wave in combination with a diode. Alternatively, a power may be supplied by means of radio communications. For example, the energization method described in Nature Biotechnology (2022) (https://doi.org/10.1038/s41587-022-01528-3) can be employed for the transdermal current-carrying patch of the present disclosure (the content of the literature is incorporated herein by reference in its entirety). According to such configuration, a positive voltage always varies in a positive region, and a negative voltage always varies in a negative region by placing a diode in series with a sine wave and the voltage does not fluctuate between a positive region and a negative region alternatingly. An energization time in the case of continuous energization can be 72 hours or shorter, 60 hours or shorter, 48 hours or shorter, 36 hours or shorter, or 24 hours or shorter to 1 hour or longer, 2 hours or longer, 3 hours or longer, 4 hours or longer, 6 hours or longer, 8 hours or longer, or 12 hours or longer, such as 1 to 72 hours, 2 to 48 hours, or 3 to 24 hours.

Drug permeation into the skin can be evaluated by a known method. For example, in the case of a drug that enters into the bloodstream when absorbed by an organism's body, for example, evaluation may be performed by incorporating a candidate drug into a transdermal current-carrying patch, adhering the patch to the skin of an organism's body, and measuring the concentration of the candidate drug (or metabolite thereof) in the blood after the elapse of a given time. For example, in the case of a drug that is integrated into the epidermis when absorbed by an organism's body, evaluation may be performed by incorporating a candidate drug into a transdermal current-carrying patch, adhering the patch to the skin of an organism's body, and measuring the concentration of the candidate drug (or metabolite thereof) in the epidermis after the elapse of a given time. Alternatively, drug permeation into the skin may be evaluated using a model experiment system instead of an organism or animal. For example, three-dimensional skin cells may be placed on a semipermeable membrane, electrodes may be provided and energized at a constant electric current, and drug permeation into the three-dimensional skin cells may be evaluated. In this case, the direction of the electric current to be applied may be reversed to evaluate as to whether or not drug permeation into the skin would be enhanced with an opposite electric current. When drug permeation into the three-dimensional skin cells is enhanced upon application of the electric potential both in the forward direction and in the reverse direction, the drug can be used for the transdermal current-carrying patch. Known and commercially available three-dimensional skin cells may be used. A person skilled in the art can verify whether drugs can be used in the present disclosure through routine experimental operations.

### Permeation evaluation system 1

A specific method for evaluating drug permeation into the skin is described. A 7 mm × 7 mm carbon cloth is coated with 80 µl of a solution of multi-walled carbon nanotubes in two separate instances and dried at 60°C. The carbon cloth is washed with pure water and further dried to prepare an electrode to be provided directly on cells. Skin-mimicking three-dimensional epidermal models (EPI-MODEL12, J-TEC) are used as the cells. Phosphatebuffered saline (PBS) is used as a reservoir solution. An Ag/AgCl electrode is introduced into the reservoir solution. Subsequently, each electrode is electrically connected to a constant voltage source meter. A drug is dissolved in an appropriate solution. The drug solution of 400 µl is added dropwise to the cells, and the carbon cloth electrode is soaked therein. First, an electrode on the cells is connected to the positive electrode, and the Ag/AgCl electrode on the reservoir solution side is connected to the negative electrode. An electric current of 20 µA is applied for 6 hours in this state. In the same manner, comparative experiment of adding 400 µl of the same drug solution dropwise to the cells is performed without the application of the electric current. A reservoir solution is sampled every given time to analyze the amount of the drug that has permeated through the cells and migrated to the reservoir solution. Analysis can be performed by HPLC analysis, mass analysis, or other means. The amount of permeation with the application of the electric current is compared with that without the application of the electric current. For the convenience of description, the amount of permeation without the application of the electric current is designated to be 100%, and the amount of permeation with the application of the electric current is described relative thereto. For example, if the amount of permeation with the application of the electric current is doubled relative to the amount of permeation without the application of the electric current (100%), such amount of permeation is described as being increased to 200%. This specific method is referred to as the " permeation evaluation system 1" herein. In an embodiment, the transdermal current-carrying patch can increase the amount of drug permeation with the application of the electric current to 110% or more, 115% or more, 120% or more, 130% or more, 140% or more, 150% or more, 200% or more, 250% or more, or 300% or more, such as 350% or more, relative to the amount of permeation without the application of the electric current (100%), when evaluated by the permeation evaluation system 1.

In an embodiment, the present disclosure provides a screening method to identify an identical drug that can be permeated into the skin through both the cathode electrode and the anode electrode with the use of the transdermal current-carrying patch. The screening method comprises:
(i) a step of placing three-dimensional skin cells on a semipermeable membrane, placing a drug-containing solution on the three-dimensional skin cells, placing a first electrode on the three-dimensional skin cells, and placing a second electrode under or within the semipermeable membrane;
(ii) a step of applying an electric potential at a constant electric current in the first direction and evaluating permeation of a drug into the three-dimensional skin cells;
(iii) a step of applying an electric potential at a constant electric current in the second direction and evaluating permeation of a drug into the three-dimensional skin cells; and
(iv) when permeation of the drugs into the three-dimensional skin cells is facilitated upon application of the electric potential both in the first direction and in the second direction, a step of identifying the drug as an identical drug that can be permeated into the skin through both the cathode electrode and the anode electrode with the use of the transdermal current-carrying patch. When permeation of a drug into the skin is enhanced upon application of the electric potential in the first direction compared with the case without application of the electric potential, permeation of the drug into the skin may be evaluated as being facilitated. Further, when permeation of a drug into the skin is enhanced upon application of the electric potential in the second direction compared with the case without application of the electric potential, permeation of the drug into the skin may be evaluated as being facilitated. Drug permeation into the skin can be examined by sampling the semipermeable membrane after energization.

In an embodiment, the present disclosure provides a method for producing a transdermal current-carrying patch. The production method comprises:
(i) a step of placing three-dimensional skin cells on a semipermeable membrane, placing a drug-containing solution on the three-dimensional skin cells, placing a first electrode on the three-dimensional skin cells, and placing a second electrode under or within the semipermeable membrane;
(ii) a step of applying an electric potential at a constant electric current in the first direction evaluating permeation of a drug into the three-dimensional skin cells;
(iii) a step of applying an electric potential at a constant electric current in the second direction and evaluating permeation of a drug into the three-dimensional skin cells;
(iv) when permeation of the drugs into the three-dimensional skin cells is facilitated upon application of the electric potential both in the first direction and in the second direction, a step of identifying the drug as an identical drug that can be permeated into the skin through both the cathode electrode and the anode electrode using the transdermal current-carrying patch; and
(v) a step of incorporating the drugs identified in step (iv) into the transdermal current-carrying patch. When permeation of a drug into the skin is enhanced upon application of the electric potential in the first direction compared with the case without application of the electric potential, permeation of the drug into the skin may be evaluated as being facilitated. Further, when permeation of a drug into the skin is enhanced upon application of the electric potential in the second direction compared with the case without application of the electric potential, permeation of the drug into the skin may be evaluated as being facilitated. Drug permeation into the skin can be examined by sampling the semipermeable membrane after energization. It is not necessary to repeat steps (i) to (iv) every time when producing a transdermal current-carrying patch, and once the drug of interest is identified in step (iv), steps (i) to (iv) may be omitted when implementing step (v).

In an embodiment, the transdermal current-carrying patch of the present disclosure does not comprise a conductive layer between an electrode and the skin. In other words, in one embodiment, a transdermal current-carrying patch that comprises a conductive layer between an electrode and the skin is excluded from the scope of the transdermal current-carrying patch of the present disclosure.

According to the present disclosure, a target compound, such as a drug, can be delivered to a subject more efficiently, compared with the use of conventional iontophoresis patches. With the use of conventional iontophoresis patches, it was impossible to cause an identical drug to be permeated into the skin through both the anode electrode and the cathode electrode. To the best of the present inventor's knowledge, no attempts to cause an identical drug to be permeated into the skin through both the anode electrode and the cathode electrode have been reported using conventional iontophoresis technology or conventional iontophoresis patches. When a drug is delivered using an iontophoresis patch, according to common general knowledge, it was believed that the drug would be delivered into an organism's body by electric repulsion. For example, a prior-art literature reports that an iontophoresis patch delivers a drug into an organism's body by electric repulsion (e.g., Patent Literature 3). However, the present inventor demonstrated surprisingly, and against common general knowledge, that an identical drug can be permeated into the skin simultaneously through both the anode electrode and the cathode electrode. Further, when using a positively-charged drug compound or when using a negatively-charged drug compound, drug permeation was facilitated. This can reduce dead weight or dead space of the transdermal current-carrying patch and make use of both electrodes. Further, the area of the electrode involved in drug delivery can be increased, compared with conventional iontophoresis patches. In addition, a step of producing a transdermal current-carrying patch can be simplified because, for example, it is not necessary to prepare two different types of drug layers.

### Examples

The transdermal current-carrying patch of the present disclosure is described in greater detail with reference to the following examples. However, it should be noted that these examples are provided for illustrative purposes only, and the present disclosure is not limited thereto in any way.

### Example 1. Evaluation of electro-osmosis (electro-permeation) of lidocaine hydrochloride

A 7 mm × 7 mm carbon cloth (Toyo Corporation) was coated with 80 µl of a solution of multi-walled carbon nanotubes in two separate instances and dried at 60°C. The carbon cloth was washed with pure water and further dried at 60°C to produce an electrode (carbon cloth electrode) to be provided directly on cells. Skin-mimicking three-dimensional epidermal models (EPI-MODEL12, J-TEC) were used as the cells. PBS was used as a reservoir solution and an Ag/AgCl electrodes (BAS) were introduced into the reservoir solution. Lidocaine hydrochloride was dissolved to 10 mg/ml in PBS/20% ethanol (pH 6.8). Since lidocaine has pKa of 7.9, lidocaine is positively charged at a pH of 6.8. The lidocaine hydrochloride solution (400 µl) was added dropwise to the cells, and the carbon cloth electrode was soaked in the solution while avoiding contact with the cells. Subsequently, in order to facilitate permeation of lidocaine hydrochloride by electric repulsion, the electrode on the cells was connected to the positive electrode, the Ag/AgCl electrode on the reservoir solution side was connected to the negative electrode, and the power supply was turned on to allow an electric current of 20 µA to flow for 6 hours with the use of the constant voltage source meter (type 2401; Tektronix, Inc.). The test was performed in the same manner wherein 400 µl of the lidocaine hydrochloride solution was added dropwise to the cells and without the application of the electric current. The reservoir solution was sampled every given period from the system with the application of the electric current and from the system without the application of the electric current to analyze the amount of lidocaine that had permeated through the cells and migrated to the reservoir solution by HPLC.

The results demonstrate that the amount of lidocaine that had migrated to the reservoir solution side; i.e., the amount of lidocaine that had permeated through the cells and migrated to the reservoir solution side, was increased in the system with the application of an electric current of 20 µA, compared with the system without the application of the electric current. More specifically, the amount of permeation was increased to 206% in the system with the application of an electric current of 20 µA for 6 hours, relative to the system without the application of the electric current (100%).

Subsequently, electrodes were connected by reversing the direction of the electric current. More specifically, the electrode on the cells was connected to the negative electrode, and the Ag/AgCl electrode on the reservoir solution side was connected to the positive electrode. Other conditions were the same as described above and an electric current of 20 µA was allowed to flow for 6 hours in the same manner except for the conditions described above, and the amount of lidocaine permeation was analyzed. Although it was presumed that, by connecting the electrodes in this manner, the amount of lidocaine permeation would be reduced due to electrical induction when an electric current is applied, contrary to such expectation, the amount of lidocaine permeation was increased in a system applying an electric current of 20 µA, compared with a system without applying an electric current. More specifically, the amount of permeation was increased to 234% when applying an electric current of 20 µA for 6 hours, compared with the system without applying the electric current (100%). Further, the test was performed by changing the electric current from 20 µA to 50 µA. The results demonstrate that the amount of permeation was increased to 357% in the system applying an electric current of 50 µA for 6 hours, compared with a system without applying an electric current (100%).

### Example 2. Evaluation of electro-osmosis of lidocaine

Evaluation was performed in the same manner except for the use of a lidocaine solution (pH 9.3) instead of the lidocaine hydrochloride solution (pH 6.8) used in Example 1. Since lidocaine has pKa of 7.9, lidocaine is not substantially charged at a pH of 9.3. In the same manner as in Example 1, the electrode on the cells was connected to the positive electrode, and the Ag/AgCl electrode on the reservoir solution side was connected to the negative electrode. Subsequently, an electric current of 20 µA was applied for 6 hours. Another experiment was performed in the same manner by adding 400 µl of the lidocaine hydrochloride solution dropwise to the cells without applying an electric current. A reservoir solution was sampled every given time to analyze by HPLC the amount of lidocaine that has permeated through the cells and migrated to the reservoir solution. The results demonstrate that the amount of lidocaine permeation did not change with or without the application of an electric current of 20 µA. More specifically, the amount of permeation measured in a system applying an electric current of 20 µA for 6 hours was 98%, relative to that measured in a system without applying an electric current (100%) and the amount of permeation was substantially equivalent therebetween.

### Example 3. Evaluation of electro-osmosis of ketoprofen

Evaluation was performed in the same manner except for the use of a ketoprofen solution (pH 6.6) instead of the lidocaine hydrochloride solution (pH 6.8) used in Example 1. Since ketoprofen has pKa of 3.9, ketoprofen is negatively charged at a pH of 6.6. In the same manner as in Example 1, the electrode on the cells was connected to the positive electrode, and the Ag/AgCl electrode on the reservoir solution side was connected to the negative electrode. Subsequently, an electric current of 20 µA was applied for 4 hours. Another experiment was performed in the same manner by adding 400 µl of the ketoprofen solution dropwise to the cells without the application of an electric current. A reservoir solution was sampled every given time to analyze by HPLC the amount of ketoprofen that has permeated through the cells and migrated to the reservoir solution. As a result, while it was presumed that the amount of ketoprofen permeation would be reduced due to electrical induction when applying an electric current due, contrary to such expectation, the amount of ketoprofen permeation was increased in a system applying an electric current of 20 µA, compared with a system without applying an electric current. More specifically, the amount of permeation was increased to 306% in a system applying an electric current of 20 µA for 2 hours, compared with a system without applying an electric current (100%).

In order to reverse the direction of the electric current, subsequently, an electrode on the cells was connected to the negative electrode, the Ag/AgCl electrode on the reservoir solution side was connected to the positive electrode, and an electric current of 20 µA was applied for 24 hours. Another experiment was performed in the same manner by adding 400 µl of the ketoprofen solution dropwise to the cells without applying an electric current. A reservoir solution was sampled every given time to analyze by HPLC the amount of ketoprofen that has permeated through the cells and migrated to the reservoir solution. The results demonstrate that the amount of ketoprofen permeation was increased in a system applying an electric current of 20 µA, compared with a system without applying an electric current. More specifically, the amount of permeation measured in a system applying an electric current of 20 µA for 2 hours was increased to 231%, relative to that measured in a system without applying an electric current (100%).

### Example 4. Evaluation of electro-osmosis of procaine hydrochloride

Evaluation was performed in the same manner except for the use of a procaine hydrochloride solution (pH 7.0) instead of the lidocaine hydrochloride solution (pH 6.8) used in Example 1. Since procaine hydrochloride has pKa of 8.9, procaine hydrochloride is positively charged at a pH of 7.0. In the same manner as in Example 1, the electrode on the cells was connected to the positive electrode, and the Ag/AgCl electrode on the reservoir solution side was connected to the negative electrode. Subsequently, an electric current of 20 µA was applied for 6 hours. Another experiment was performed in the same manner by adding 400 µl of the procaine hydrochloride solution dropwise to the cells without applying an electric current. A reservoir solution was sampled every given time to analyze by HPLC the amount of procaine hydrochloride that has permeated through the cells and migrated to the reservoir solution. The results demonstrate that the amount of procaine hydrochloride permeation was increased in a system applying an electric current of 20 µA, compared with a system without applying an electric current. More specifically, the amount of permeation was increased to 157% in a system applying an electric current of 20 µA for 6 hours, compared with a system without applying an electric current (100%).

Subsequently, electrodes were connected to reverse the direction of the electric current. More specifically, the electrode on the cells was connected to the negative electrode, and the Ag/AgCl electrode on the reservoir solution side was connected to the positive electrode. In the same manner, an electric current of 20 µA was applied for 24 hours, and the amount of procaine hydrochloride permeation was analyzed. As a result, while it was presumed that the amount of procaine hydrochloride permeation would be reduced due to electrical induction when applying an electric current, contrary to such expectation, the amount of procaine hydrochloride permeation was increased in a system applying an electric current of 20 µA, compared with a system without applying an electric current. More specifically, the amount of permeation was increased to 284% in a system applying an electric current of 20 µA for 6 hours, compared with a system without applying an electric current (100%).

### Example 5. Evaluation of electro-osmosis of tetracaine hydrochloride

Evaluation was performed in the same manner except for the use of a tetracaine hydrochloride solution (pH 6.9) instead of the lidocaine hydrochloride solution (pH 6.8) used in Example 1. Since tetracaine hydrochloride has pKa of 8.4, procaine hydrochloride is positively charged at a pH of 7.0. In the same manner as in Example 1, the electrode on the cells was connected to the positive electrode, and the Ag/AgCl electrode on the reservoir solution side was connected to the negative electrode. Subsequently, an electric current of 20 µA was applied for 6 hours. Another experiment was performed in the same manner by adding 400 µl of the tetracaine hydrochloride solution dropwise to the cells without applying an electric current. A reservoir solution was sampled every given time to analyze by HPLC the amount of tetracaine hydrochloride that has permeated through the cells and migrated to the reservoir solution. The results demonstrate that the amount of tetracaine hydrochloride permeation was increased in a system applying an electric current of 20 µA, compared with a system without applying an electric current. More specifically, the amount of permeation was increased to 138% in a system applying an electric current of 20 µA for 6 hours, compared with a system without applying an electric current (100%).

Subsequently, electrodes were connected to reverse the direction of the electric current. More specifically, the electrode on the cells was connected to the negative electrode, and the Ag/AgCl electrode on the reservoir solution side was connected to the positive electrode. In the same manner, an electric current of 20 µA was applied for 6 hours, and the amount of tetracaine hydrochloride permeation was analyzed. As a result, while it was presumed that the amount of tetracaine hydrochloride permeation would be reduced due to electrical induction when applying an electric current, contrary to such expectation, the amount of tetracaine hydrochloride permeation was increased in a system applying an electric current of 20 µA, compared with a system without applying an electric current. More specifically, the amount of permeation was increased to 115% in a system applying an electric current of 20 µA for 6 hours, compared with a system without applying an electric current (100%).

### Example 6. Production example of transdermal current-carrying patch 1

The materials indicated below were prepared to produce the transdermal current-carrying patch 1.

### Electrode body 10:

Carbon fiber (Toho Tenax) comprising multi-walled carbon nanotubes (Baytube) supported thereon was used as a material to produce (prepare) the electrode body 10 having the configuration shown in Figure 1. Carbon nanotubes manufactured by Meijo Nano Carbon Co., Ltd. may be used, although carbon nanotubes are not particularly limited thereto. Further, carbon fiber manufactured by Toray Industries, Inc. may be used, although carbon fiber is not particularly limited thereto. The thickness of the electrode body 10 was 0.3 mm. The area of the anode electrode 11 and that of the cathode electrode 12 were 1.7 cm² each. 4-Isopropylaminodiphenylamine and glucose dehydrogenase were supported on the anode electrode 11 as catalysts. Carbon fiber comprising multi-walled carbon nanotubes and polytetrafluoroethylene supported thereon was used for the cathode electrode 12. Iron phthalocyanine (Tokyo Chemical Industry Co., Ltd.) was supported as a catalyst. The lead 13 was produced using carbon fiber. The anode electrode 11 and the cathode electrode 12 were connected to the lead 13 via heat fusion.

### Conductive part 20:

A 50 mM McIlvaine buffer solution (300 µl, pH 5) and a 200 mM glucose solution were added to a polyurethane sponge (product name: Sofras, manufactured by Aion Co., Ltd.), and the sponge was dried to produce (prepare) the conductive part 20. The thickness of the conductive part 20 was 1 mm.

### Adhesive layer 30:

The adhesive layer 30 was prepared using a two-sided adhesive tape for medical use (3M Japan Limited) as a two-sided tape for skin. The thickness of the adhesive layer 30 was 0.16 mm.

### Separator 40:

The separator 40 having the configuration shown in Figure 1 was prepared using polyester as a material. A one-sided polyethylene-coated paper, polypropylene, or the like may be used as the separator 40.

### Surface film 50:

The surface film 50 having the configuration shown in Figure 1 was prepared using a polyvinyl chloride film as a material.

After the materials indicated above were prepared, the electrode body 10, the conductive parts 20, the adhesive layer 30, the separator 40, and the surface film 50 were assembled in the order and in the arrangement shown in Figure 1 to prepare a large number of transdermal current-carrying patches 1. The electric current density in the electric circuit of the patch of Example 6 was measured using the ALS electrochemical analyzer 814D (BAS) and the results of measurements are shown in Table 1 below. The electric circuit composed of the anode electrode 11, the cathode electrode 12, the conductive parts 20 A and 20B, and the lead 13 was connected to the resistance shown in Table 1, and the voltage of said electric circuit was measured using the analyzer indicated above (voltmeter). The electric current values obtained based on the voltage measured and the resistance were divided by the area (approximately 1.7 cm²) of the conductive parts 20 (each of 20A and 20B) to determine the electric current density. The "current density" shown in Table 1 is the value measured approximately 10 minutes after the addition of a substrate-containing solution, no significant reduction was observed with the elapse of time, and the value was merely slightly reduced after the elapse of 60 minutes.

**[Table 1 ]**

| Resistance R (kΩ) | Voltage (V) | Current density (µA/cm²) |
|---|---|---|
| 20 | 0.27 | 8 |
| 10 | 0.23 | 14 |
| 5 | 0.20 | 23 |
| 2 | 0.14 | 43 |
| 1 | 0.11 | 63 |

The transdermal current-carrying patch 1 was configured such that the electric current density of the micro electric current flowing when the electric circuit of the patch was connected to a resistance of 10 kΩ was in a range of 10 µA/cm² to 30 µA/cm² at a time point up to 10 minutes after the addition of water to the transdermal current-carrying patch. More specifically, the patch was configured such that the micro electric current flowing to the given site of the subject was 5 µA/cm² or more after the elapse of at least 1 hour. The open circuit voltage applied by the transdermal current-carrying patch was approximately 300 mV.

### Example 7. Production example of transdermal current-carrying patch 2

As an alternative to the power supply, the transdermal current-carrying patch can be combined with a button battery instead of an enzyme battery. The transdermal current-carrying patch 2 comprising a button battery was produced using the materials indicated below in accordance with the procedure described below. First, the materials indicated below were prepared.

Carbon fiber comprising multi-walled carbon nanotubes supported thereon was used for the anode electrode 11 and the cathode electrode 12, and the carbon fiber was electrically connected to an alkaline button battery (1.5V, LR44, Panasonic Corporation). The anode electrode 11 was connected to the cathode electrode 12 via a stainless steel wire (equivalent to the lead 13). Other materials were assembled in the same manner as in Example 6 to construct transdermal current-carrying patches. Power generation was initiated with the addition of a 100 mM potassium phosphate buffer (pH 7) to the sponge of the conductive parts 20. The electric current density in the electric circuit of the patch of Example 3 was as shown in Table 2 below. The "current density" shown in Table 2 is the value measured approximately 10 minutes after the connection of the electric circuit, no significant reduction was observed with the elapse of time, and the value was merely slightly reduced after the elapse of 60 minutes.

**[Table 2]**

| Resistance R (kΩ) | Voltage (V) | Current density (µA/cm²) |
|---|---|---|
| 20 | 1.20 | 35 |
| 10 | 0.97 | 57 |
| 5 | 0.74 | 87 |
| 2 | 0.48 | 142 |
| 1 | 0.41 | 242 |

The transdermal current-carrying patch 2 combined with a the button battery was configured such that the electric current density of a micro electric current flowing when the electric circuit of the patch was connected to a resistance of 10 kΩ was in a range of 50 µA/cm² to 100 µA/cm² at a time point up to 10 minutes after the initiation of energization. More specifically, the patch was configured such that the micro electric current flowing to the given site of the subject was 30 µA/cm² after the elapse of at least 1 hour. When the electric circuit was connected to a resistance of 5 kΩ, the electric current was found to flow at a current density of 87 µA/cm² and when the electric circuit was connected to a resistance of 1 kΩ, the electric current was found to flow at a current density of 242 µA/cm². More specifically, the electric current density of the tested patch was lower than 500 µA/cm², and it was confirmed that there was no risk of skin irritation for the tested patch.

### Example 8. Application of transdermal current-carrying patch to the skin

The transdermal current-carrying patch 1 prepared above (Example 6) is applied to the skin of the subject. The area of each electrode is 1.7 cm². The separator 40 is removed to expose the adhesive layer 30, and the patch is then adhered to the skin of the subject. According to the Handbook for electric installation and safety and sanitation thereof, Vol. 4, the Ship's Electric Installation Contractors' Association of Japan, the electric resistance of the skin is approximately 10 kΩ on average. As such, with the use of the transdermal current-carrying patch 1 described above, an electric current is computed to flow at 14 µA/cm² and if the area of the electrode is 1.4 cm² or larger, an electric current of 20 µA or higher is computed to flow. This indicates that including a drug, such as procaine hydrochloride and the like, in the conductive part 20 can facilitate permeation of the drug, such as procaine hydrochloride and the like.

### Industrial Applicability

According to the present disclosure, a compound of interest, such as a drug, can be delivered to a subject using iontophoresis technology.

This description cites documents including literatures, patent literatures, and the manufacturers' instructions and the like. While the disclosures of such documents are not regarded as being related to patentability of the present invention, such disclosures are incorporated herein by reference in their entirety. More specifically, all of the reference documents are incorporated herein by reference as in the case in which each of the documents is specifically and individually indicated to be incorporated herein by reference.

### Reference Signs List

1: Transdermal current-carrying patch
10: Electrode body
11: First electrode (anode electrode)
12: Second electrode (cathode electrode)
13: Lead
14: Enzyme
15: Mediator
20: Conductive part
20A: First conductive layer
20B: Second conductive layer
30: Adhesive layer
31: Opening
32: Opening
33: Part between the opening 31 and the opening 32 (insulated part)
40: Separator
41: Opening
42: Opening
50: Surface film 50
51: Window part

## Claims

1. A transdermal current-carrying patch to be brought into contact with the skin to allow a drug to be permeated into the skin,
wherein the transdermal current-carrying patch comprises an electrode body, a conductive part, an adhesive layer, a separator, and a surface film,
the electrode body comprises a first electrode and a second electrode,
the conductive part comprises a first conductive layer and a second conductive layer,
the separator covers the adhesive layer and is to be removed when using the transdermal current-carrying patch,
the adhesive layer is for adhering the transdermal current-carrying patch to the target skin and insulates the first conductive layer from the second conductive layer,
the first conductive layer comprises a first electrolyte, a first buffer, and a drug,
the second conductive layer comprises a second electrolyte, a second buffer, and a drug, and
the first electrode, the second electrode, and the conductive part are connected to a power supply,
wherein, when the transdermal current-carrying patch is brought into contact with the skin, an electric potential is applied from the power supply to enable power distribution among the first electrode, the skin, and the second electrode, thereby allowing an electric current to flow, and
wherein, the drug comprised in the first conductive layer is identical to the drug comprised in the second conductive layer.

2. The transdermal current-carrying patch according to claim 1, wherein permeation of both the drug comprised in the first conductive layer and the drug comprised in the second conductive layer into the skin are facilitated, compared with the case where an electric current does not flow.

3. The transdermal current-carrying patch according to claim 1 or 2, wherein the drug is a positively-charged drug.

4. The transdermal current-carrying patch according to claim 1 or 2, wherein the drug is a negatively-charged drug.

5. A screening method to identify an identical drug that can be permeated into the skin through both the cathode electrode and the anode electrode by using a transdermal current-carrying patch, said screening method comprising:
(i) a step of placing three-dimensional skin cells on a semipermeable membrane, placing a drug-containing solution on the three-dimensional skin cells, placing a first electrode on the three-dimensional skin cells, and placing a second electrode under or within the semipermeable membrane;
(ii) a step of applying an electric potential at a constant electric current in the first direction and evaluating permeation of a drug into the three-dimensional skin cells;
(iii) a step of applying an electric potential at a constant electric current in the second direction and evaluating permeation of a drug into the three-dimensional skin cells; and
(iv) when permeation of the drugs into the three-dimensional skin cells is facilitated upon application of the electric potential both in the first direction and in the second direction, a step of identifying the drug as an identical drug that can be permeated into the skin through both the cathode electrode and the anode electrode by using the transdermal current-carrying patch,
wherein, when permeation of a drug into the skin is enhanced upon application of the electric potential in the first direction compared with the case without application of the electric potential, permeation of the drug into the skin is evaluated as being facilitated, and
when permeation of a drug into the skin is enhanced upon application of the electric potential in the second direction compared with the case without application of the electric potential, permeation of the drug into the skin is evaluated as being facilitated.

6. A method for producing a transdermal current-carrying patch comprising:
(i) a step of placing three-dimensional skin cells on a semipermeable membrane, placing a drug-containing solution on the three-dimensional skin cells, placing a first electrode on the three-dimensional skin cells, and placing a second electrode under or within the semipermeable membrane;
(ii) a step of applying an electric potential at a constant electric current in the first direction and evaluating permeation of a drug into the three-dimensional skin cells;
(iii) a step of applying an electric potential at a constant electric current in the second direction and evaluating permeation of a drug into the three-dimensional skin cells;
(iv) when permeation of the drug into the three-dimensional skin cells is facilitated upon application of the electric potential both in the first direction and in the second direction, a step of identifying the drug as an identical drug that can be permeate into the skin through both the cathode electrode and the anode electrode by using the transdermal current-carrying patch; and
(v) a step of incorporating the drug identified in step (iv) into the transdermal current-carrying patch.
